# EUROPEAN PATENT SPECIFICATION

(11) **EP 2 938 742 B1**
(45) Date of publication and mention of the grant of the patent: **15.02.2017**
(21) Application number: 12821101.8
(22) Date of filing: 28.12.2012
(51) Int. Cl.: C12Q 1/68

(54) **METHOD FOR COMPLETE TRACKING OF A SET OF BIOLOGICAL SAMPLES CONTAINING DNA OR RNA THROUGH MOLECULAR BARCODE IDENTIFICATION DURING LABORATORIAL WORKFLOW AND KIT FOR COLLECTING BIOLOGICAL SAMPLES CONTAINING DNA OR RNA**
VERFAHREN ZUR VOLLSTÄNDIGEN VERFOLGUNG EINES SATZES VON BIOLOGISCHEN PROBEN MIT DNA ODER RNA DURCH MOLEKULARE STRICHCODEIDENTIFIKATION WÄHREND EINES LABORARBEITSABLAUFS UND KIT ZUR SAMMLUNG VON BIOLOGISCHEN PROBEN MIT DNA ODER RNA
PROCÉDÉ DE DÉTECTION COMPLÈTE D'UN ENSEMBLE D'ÉCHANTILLONS BIOLOGIQUES CONTENANT DE L'ADN OU DE L'ARN PAR L'IDENTIFICATION DE CODES À BARRES MOLÉCULAIRES PENDANT LE FLUX DE TRAVAUX DE LABORATOIRE, ET TROUSSE POUR PRÉLEVER DES ÉCHANTILLONS BIOLOGIQUES CONTENANT DE L'ADN OU DE L'ARN

(43) Date of publication of application: 04.11.2015
(73) Proprietor: Fleury S.A., São Paulo - SP, CEP: 04344-903 (BR)
(72) Inventor: MITNE NETO, Miguel, Itaim São Paulo - SP (BR); MYASHIRO, Kozue, São Paulo - SP (BR); DOS SANTOS, Marcos Tadeu, São Paulo - SP (BR)
(74) Representative: Michalski Hüttermann & Partner Patentanwälte mbB
(86) International application number: PCT/BR2012/000548
(87) International publication number: WO 2014/100866

(56) References cited:
- WO-A1-2010/115154
- WO-A2-2012/162267
- US-A1- 2010 227 329
- DANIEL N FRANK: "BARCRAWL and BARTAB: software tools for the design and implementation of barcoded primers for highly multiplexed DNA sequencing", BMC BIOINFORMATICS, vol. 10, no. 1, 1 January 2009 (2009-01-01), page 362, XP055003933, ISSN: 1471-2105, DOI: 10.1186/1471-2105-10-362

## Description

### • Field of the Invention

The invention relates to a method for complete tracking of multiple samples during laboratorial workflow by adding a molecular barcode to target sequences through a one-step polymerase chain reaction (PCR). This one-step PCR method for tracking multiple biological samples permits an early and reliable identification of biological samples requiring only a very small amount of sample.

### • Background of the invention

The development of the sequencing analysis technology in the field of molecular biology has shown to be a key tool in the researching of all branches of biological research. More specifically in the medical area, sequencing analysis plays an important role in the diagnosis and prognosis of genetic disorders. These diagnoses have been performed much more effectively over time with the development of newer and faster sequencing methodologies and equipments.

The automated Sanger sequencing has been the most important sequencing method in the scientific area for almost two decades (Metzker, M. Sequencing technologies - the next generation. Nature reviews Genetics (11). 2010). The completion of the only finish-grade human genome sequence, among other relevant accomplishments, was possible due to the Sanger sequencing.

Recently, new methodologies of sequencing have been developed. These technologies called next-generation sequencing (NGS) have the capacity of producing huge amounts of data in a fast and cheap manner, consisting basically of the steps of template preparation, sequencing and data analysis (Metzker, M. Sequencing technologies - the next generation. Nature reviews Genetics (11). 2010).

NGS can sequence up to billions of bases in a single day at a low cost (Pop, M. & Salzberg, S. Bioinformatics challenges of new sequencing technology. Trends Genet. 24(3). 2008). Although achieving an increasingly higher throughput is the ultimate goal of NGS technology, this increasingly higher throughput also presents considerable challenges for the technology.

In this sense, the massive amount of data produced by NGS methodologies place substantial demands on the development of more efficient routines in terms of data storage, tracking and quality control.

New findings in the medical area relate to common disorders, as cancer, phenylketonuria, among others, with mutations in the human genome. Therefore, the diagnosis and prognosis of such disorders benefits from sequencing methodologies advancements. Currently, laboratory processes involved in molecular diagnosis are highly complex and have proven to be difficult to standardize and automate (Gomah, M. et al. Modeling Complex Workflow in Molecular Diagnostics. The Journal of Molecular Diagnostics 12(1). 2010). The throughput of many samples brings an extra challenge; namely, the exchange of samples when analyzing a considerable number of experiments. Errors in tracking and in exchanging samples result in costly accidents in a clinical laboratory workflow, implications for patients, laboratories and operators.

Laboratorial tests, particularly in this biotechnological field, must therefore be performed under a high quality control system that is able to easily and accurately identify biological samples over the entire lifetime of the biological sample in the laboratory. However, particularly when considerably high numbers of samples are generated, the workflow becomes increasingly costly and complex. Therefore, large investments are necessary to maintain a structure in high quality standards.

The common identification system used in laboratories consists of labeling the sample contained in test tubes. Although this routine is very useful, the object being tracked is a tube or recipient and not specifically the analyte, the specific biological or chemical material contained by the tube, per se. Therefore, this laboratorial routine of tracking samples only by labeling the test tubes does not exclude the possibility of human mistakes during the manipulation of the samples over the laboratory workflow.

In this sense, molecular barcodes are an option that may be used in order to track analytes during molecular assay workflows, since they represent an entity with similar properties to the analyte. Molecular barcodes are, simply put, unique molecules used to identify a particular sample in a pool of samples. And in this present case may be inserted directly within the biologic material being analyzed so as to easily and rapidly identify its contents.

US 2010/0227329 A1 discloses processes of tagging a DNA target, through a two-step PCR reaction, using two different annealing temperatures for primers and barcodes and in the presence of linker molecules, such as avidin or streptavidin, to link functional molecules and core molecules.

WO 2010/115154 relates to a multi-step barcoding method directed towards target nucleic acids, wherein three primers are employed: reverse and forward amplification primers linked to a nucleotide tag and a barcode primer linked to a tag-specific portion.

WO 2012/019765 also discloses a method for combinatorial tracking of samples for parallel analysis and in particular a method for randomly mixing members of different libraries, each with a unique identifier. It is also disclosed that the insertion of the specific primers and barcodes occurs in different annealing temperatures; hence, making use of a two-step PCR reaction.

These documents show strategies for barcode insertions after the genetic material extraction; however, the documents cited do not adequately resolve the aforementioned problems regarding the elimination of errors as characterized by the traditional labeling of test tube collections, due in large part to the number of steps and subsequent transfers of the sample material before being reliably labeled.

In this sense, it is crucial to develop methods aiming at reducing the number of steps in labeling sample material over the course of the laboratory workflow, particularly when preparing and tagging samples for routine sequencing analyses. Ideally, these methods would allow for complete sample tracking and concomitantly reduce the chances for error.

Accordingly, it follows that the present invention discloses a novel comprehensive single-step complete sample tracking process, allowing for biological sample tracking from the moment of the sample collection through the final analysis report, in which the genetic material extraction step can be omitted and in which the barcode insertion occurs in a single step polymerase chain reaction, as defined in the appended claims.

In this invention, barcodes and the universal primers are in contact with the sample since its first collection, thus providing a complete tracking thereof from the initial introduction of biological sample collection. Furthermore, in the one-step PCR reaction detailed in the present invention, the annealing of specific primers for amplification and the annealing of adapters for barcodes insertion occur at the same temperature; hence, there is no physical or time separation between sample and barcode during the entire process.

Thus, this invention brings as a benefit over the prior art the ability to track biological samples containing genetic material from the moment of the biological materials first collection through its final analysis and this is achieved by a single step polymerase chain reaction. In other words, this invention presents a means by which biological samples are rapidly and reliably identified over the course of a laboratory workflow, minimizing chances for errors to occur, and hence improving the efficiency of the laboratory workflow and the overall research and development involving biological samples.

Finally, as an additional benefit, a very small sample amount is required according to the present invention when compared to the state of art. For example, generally, the prior art methods require around 3mL of blood through venous puncture, while the method of the present invention requires amounts as little as 2µL of blood. Therefore, it is possible to obtain the biological sample through a simple finger prick blood collection followed by contacting the blood drop with a filter paper.

The possibility of using a very small sample amount is extremely important to improve the efficiency of the laboratorial workflow, in particular with respect to clinical analysis laboratories, as a result of a reduction in the time involved in collecting blood, the costs of the analyses to be conducted, as well as also increasing patients comfort during this collection process.

### • Summary of the invention.

The present invention relates to a method of complete biological sample tracking. In particular, the analyte itself is tracked from the moment of its collection through the insertion of molecular barcodes and the amplification of the target sequence. The method allows for complete and reliable biological sample tracking, eliminating the chances of misidentifying the collected biological samples.

In certain embodiments, the invention discloses a method of complete sample tracking since its collection, wherein the molecular barcodes insertion occurs simultaneously to the amplification of the target sequence in a single PCR step.

In a certain embodiment, a sample is placed in a tube comprising a first pair of oligonucleotide primers consisting of a molecular barcode binding to an universal primer. The following step comprises the removal of an aliquot and placing it in the PCR tube comprising a second pair of oligonucleotide primers consisting in a specific primer binding to a universal primer region, wherein the specific primers are specific for a target region of the biological sample and the universal primer regions are complementary to the universal primers of the first pair of oligonucleotides. Then, the amplification of the target regions occurs concomitantly to the barcode insertion in the same annealing temperature through a one step PCR, without the need of steps of initial extraction or purification prior to the sequencing analysis. However, if preferred, initial extraction or the purification steps can be performed,

Such strategy can be applied, but are not restricted, to diagnostic methods involving Sanger and next-generation sequencing (NGS), as for example, but not restricted to, specific or captured amplicons, transcriptomes, exomes, miRNomes and genomes.

The following figures are for illustrative purposes only and do not limit the invention described above in any way.

### • Brief description of the drawings.

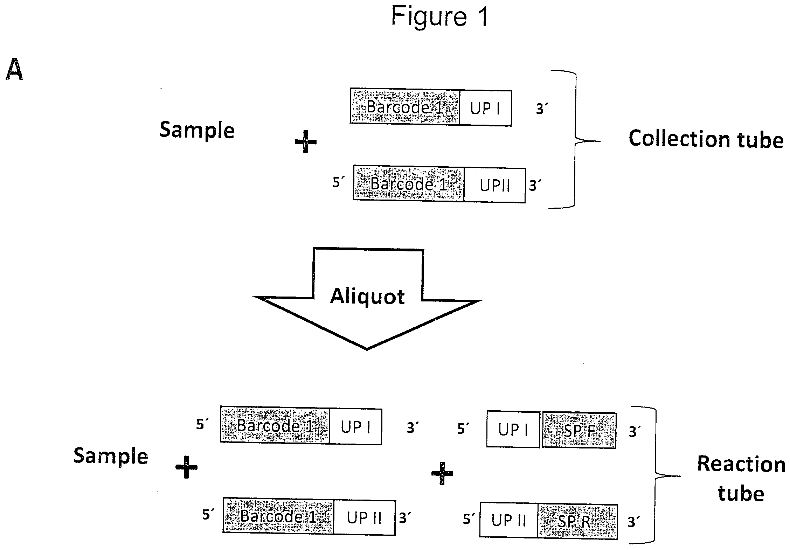

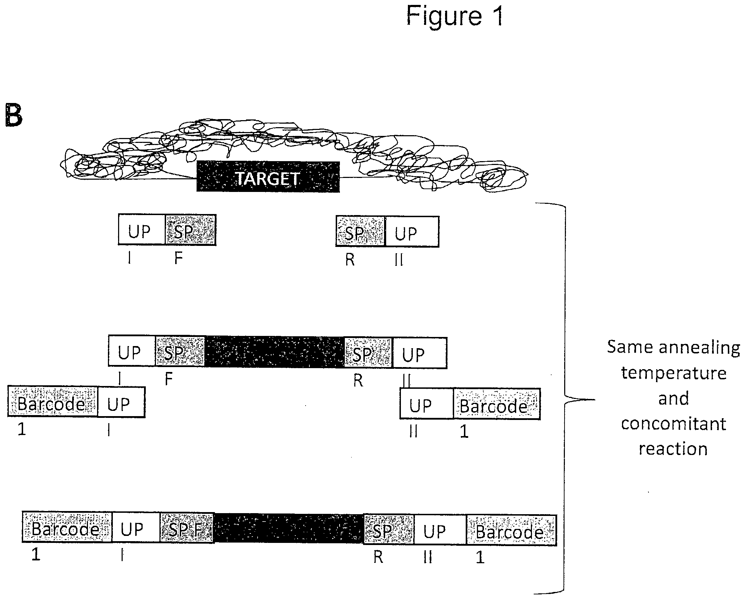

**Figure 1****.** Example of a sample tagged with barcode 1 for Sanger sequencing. Differences regarding oligonucleotides used for NGS are described in the text.
   A) Sample barcoding workflow. The sample is collected in a tube containing oligonucleotides with the barcodes and universal primers I(UP I) and universal primers II (UP II). An aliquot of this mix (sample + barcode) is added to the PCR reagents (here shown only by the specific primers (SP F or SP R) with universal primers) making it possible to amplify the target region and insert the barcodes in a single PCR reaction.
   B) Target region assembly. The target region is amplified by the annealing of the specific primers, which contain universal primers. The annealing of specific primers and universal primers in tandem with barcode sequences occurs at the same temperature and time, differently from the state of art, in which the annealing temperatures are different and the PCR occurs in two steps.

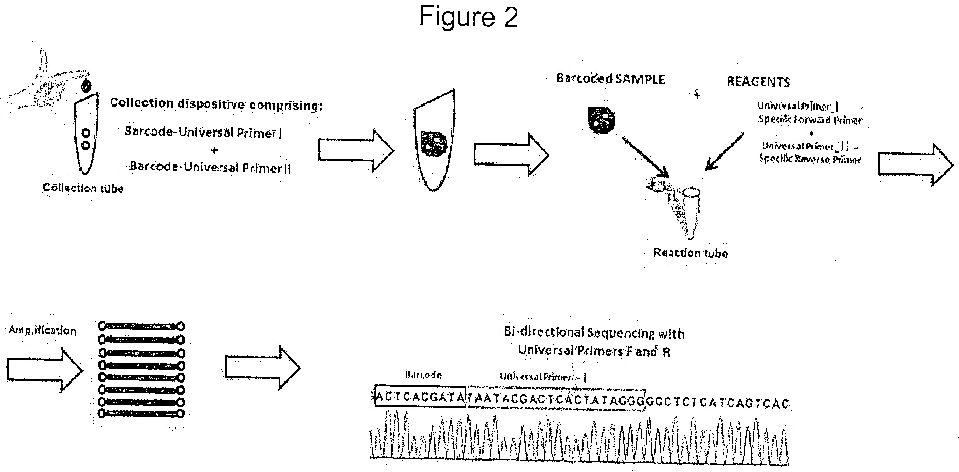

**Figure2****.** Sanger sample barcoding overview: a pair of oligonucleotides (barcode-universal primer **F** and **R**) is already inside of the collection dispositive. Following the diagnostic workflow, an aliquot of the sample containing the barcodes is used for amplification. This step allows the barcode's insertion on both extremities of the target region. Bi-directional sequencing with universal primers or with specific primers can detect target region variations and concomitantly identify the respective barcode.

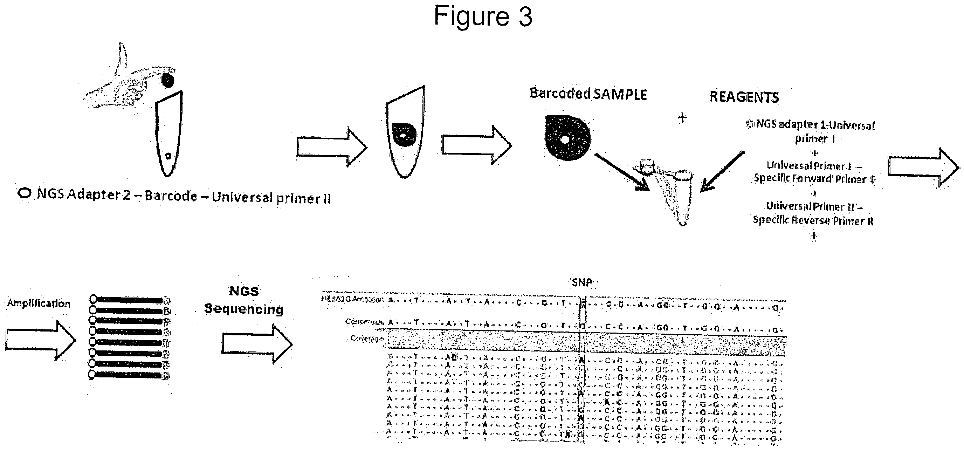

**Figure 3****.** Sample barcoding preparation for next generation sequencing. The method uses an oligonucleotide (here exemplified as NGS adapter 2 - barcode-universal primer II), which will be inserted into the generated target region. As it is described for Sanger Sequencing barcoding, the method uses a single step PCR reaction. Besides the specific primers F and R, one has to use an oligonucleotide containing an NGS adapter 1-universal primer sequence, in order to have a target region that can be sequenced in a NGS system. Only corrected target fragments can be sequenced, "NGS 1" and "NGS 2" adaptors need to be in the correct position and frame.

### • Detailed description.

The present invention describes a method to prepare a plurality of nucleic acids target sequences of a biological sample to, first, facilitate the analysis of the biological sample through the use of molecular methods, mainly by sequencing methodologies, and, second, to identify rapidly and reliably each biological sample in a sample pool.

In a preferred embodiment the oligonucleotides comprising the specific primer and universal primer as well as the barcodes and universal primer, respectively, are linked to the target sequence in a one step polymerase chain reaction (PCR), in which the annealing temperature is the same for both oligonucleotides.

Therefore, the embodiments disclosed by the present invention, as well the advantages over the state of art must be read concomitantly with the examples and figures also disclosed by the present invention.

The term "universal primers" refers to any sequences known to anneal to target sequences that do not interfere in the amplification of targeted genome/transcriptome/exome/miRNome region, either among themselves.

The terms "one step PCR" means a polymerase chain reaction in which the annealing temperature of the two sets of primers (barcode and universal primers as well as specific primers and universal primers) is the same and the reactions occurs at the same time.

The term "collection, tube" means the first recipient where the biological sample is conditioned after its collection.

The term "barcode" means an oligonucleotide present in a nucleic acid sequence in order to identify it. Such identification is made through different laboratorial approaches.

In one embodiment of the invention, it is provided for a method that allows for the complete tracking of a set of biological samples containing DNA or RNA through molecular barcode identification during laboratorial workflow comprising the following steps:
i) contacting one oligonucleotide comprising molecular barcodes and universal primers with the biological samples containing DNA or RNA in a collection means, thus forming mix A;
ii) contacting mix A with a PCR mix comprising one oligonucleotide comprising specific primers and universal primers, thus forming mix B;
iii) amplifying the target region of the biological samples containing DNA or RNA and inserting the molecular barcodes in one or both ends of said target region in a single and concomitant step by performing a unique multiplex one step PCR in mix B;
iv) analyzing the product obtained in step iii) by any molecular technology.

In a preferred embodiment, the molecular barcodes are synthesized, or generated through molecular biology methodologies, in tandem with the universal primers.

In a further embodiment, the molecular barcodes are from 4 to 30 nucleotides long, preferably composed between 8 to 12 nucleotides and most preferably 10 nucleotides long.

In another embodiment, the volume of said biological samples containing DNA or RNA is from 0,5µL to 5mL, more preferably from 1µL to 10µL and most preferably 2µL.

In a further embodiment, the annealing temperatures for amplification and for barcode insertion in the multiplex one step PCR of step iii) are held constant.

In a preferred embodiment, the annealing temperature is between 35°C to 80°C, more preferably between 45°C and 70°C, and most preferably 56°C.

In a further embodiment, the number of cycles of the multiplex one-step PCR of step iii) is between 10 and 60 cycles, more preferably between 20 and 50 cycles, and most preferably 45 cycles.

The method of the present invention is useful for a series of different biological samples from human or any other eukaryotic or prokaryotic organisms selected from the group consisting of blood and other fluids, paraffin or other forms of fixed tissues, cryopreserved samples, cultured cells, tissues, seeds, leaves, exudates, lavages and swabs material.

In a preferred embodiment, the biological sample containing DNA or RNA is blood.

In another embodiment, the blood is contacted with anticoagulants selected from the group consisting of EDTA, heparin or citrated-based anticoagulants.

The method of the present invention is also useful for a series of different synthetic samples that can be produced through in vivo or in vitro strategies.

In a further embodiment, the universal primers of steps i) and ii) are universal primers M13 and T7.

In a preferred embodiment, the molecular technology is a sequencing methodology.

In an even more preferred embodiment, the sequencing methodology is Sanger or Next-Generation Sequencing.

In a further embodiment, the collection means is selected form the group consisting of tubes, recipients, plates, papers, swabs and pipettes.

In a further embodiment, the molecular barcodes and universal primers are already present in the collection means in step (i).

In a further embodiment, step (i) is carried out immediately after the collection of said biological samples containing DNA or RNA.

In a further embodiment, the steps (i) and (ii) are carried out simultaneously.

In another embodiment, step (ii) is carried out after step (i) in a reaction tube or recipient.

The invention also discloses a method for complete tracking a set of biological samples containing DNA or RNA through molecular barcode identification during laboratorial workflow, comprising the following steps:
i) contacting one oligonucleotide comprising molecular barcodes and universal primers with the biological samples containing DNA or RNA in a collection means, thus forming mix A;
ii) transferring an aliquot of mix A to a reaction tube or recipient wherein said reaction tube or recipient comprises PCR mix comprising one oligonucleotide comprising specific primers and universal primers, thus forming mix B;
iii) amplifying the target region of the biological samples containing DNA or RNA and inserting the molecular barcodes in one or both ends of said target region in a single and concomitant step by performing a unique multiplex one-step PCR in mix B using the same annealing temperature for amplification and for barcode insertion;
iv) analyzing the product obtained in step (iii) by any molecular technology.

Furthermore, the invention also discloses a method for complete tracking of a set of biological samples containing DNA or DNA through molecular barcode identification during laboratorial workflow, comprising the following steps:
i) adding biological samples containing DNA or RNA to a collection means containing one oligonucleotide comprising molecular barcodes and universal primers, thus forming mix A;
ii) transferring an aliquot of mix A to a reaction tube or recipient wherein said reaction tube or recipient comprises PCR mix comprising one oligonucleotide comprising specific primers and universal primers, thus forming mix B;
iii) amplifying the target region of the biological samples containing DNA or RNA and inserting the molecular barcodes in one or both ends of said target region in a single and concomitant step by performing a unique multiplex one-step PCR in mix B using the same annealing temperature for amplification and for barcode insertion;
iv) analyzing the product obtained in step (iii) by any molecular technology.

One embodiment of the invention provides for a kit for collecting biological samples containing DNA or RNA comprising a set of collection tubes or recipients additionally comprising molecular barcode, universal primers, specific primers and PCR mix in each collection tube or recipient for use in a method as disclosed by the present invention.

In a further embodiment, the invention also provides an additional kit for collecting biological samples containing DNA or RNA comprising a first set of collection tubes or recipients comprising molecular barcodes and universal primers in each collection tube or recipient, and a second set of reaction tubes or recipients comprising specific primers and PCR mix in each reaction tube or recipient, for use in a method as disclosed by the present invention.

### Biological Samples

The method of the present invention is useful for a series of different biological samples from human, other eukaryotic and prokaryotic organisms, or synthetic sequences generated through in vivo or in vitro strategies. Target samples could be, but are not restricted to any of the following: blood and other fluids, paraffin or other forms of fixed tissues, cryopreserved samples, cultured cells, tissues, seeds, leaves, exudates, lavages and swabs material.

The present invention provides the advantage of the collection of blood through a finger prick collection followed by contact of the blood drop with a filter paper or a small volume of anti-coagulant, since the blood volume used by the methodology can be very low.

In the case of blood samples, depending on the application it may be necessary that the collection tube comprises an anticoagulant. Examples of anticoagulants that can be used are those selected from the group consisting of EDTA, heparin or citrated-based anticoagulants.

### Sanger Sequencing Workflow

Before the material collection, sample tracking is done normally with the use of numeric barcodes and individual information in a proper system. In a preferred embodiment, the sample is collected using a collection dispositive containing the molecular barcodes. In a preferred embodiment a sample aliquot (with the oligonucleotide comprising molecular barcodes + universal primers) is added to lysis reagents. In a further embodiment, after lysis, an aliquot of this mixture (with the oligonucleotide comprising molecular barcodes + universal primers) is added to the PCR mix (among the components, the oligonucleotide comprising the specific primers + universal primers). The reaction occurs with the amplification of the target region followed by barcode insertion, without the need to stop the reaction and with the same annealing temperature for both oligonuleotides.

In a further embodiment, the target region is analyzed by Sanger sequencing procedures, which can be done by specific or universal primers. Barcodes are present at the end of each target region. Using bi-directional sequencing it is possible to guarantee the barcode presence in each of the reactions (forward and reverse).

In this sense, by the end of a laboratory workflow, the technician will be able to check the unique barcode against the sample number, thus proving that the reading result pertains to the referenced individual number upon collection and identifing the sample.

A simplified overview of the entire processes is shown in figure 2.

It is also important to note that the strategy described here can be applied if a genetic material extraction is required. The use of automated processes to perform an extraction does not restrict in anyway the invention. Finally, once the genetic material is collected, the sample is ready to be tagged, or identified, with a barcode.

### Next Generation Sequencing Workflow

In a further embodiment, the sample preparation for NGS purposes uses a very similar strategy as described for Sanger sequencing. However, for some NGS applications it is necessary to insert adapters at the end of each target region, in order to have a proper sample sequencing preparation. As an example, for use in the Ion PGM system, it is necessary the presence of adapters defined as "A" and "P1". The oligonucleotides containing the adapter "A" and "P1" were synthesized in a format that the adapter is in the barcode 5' end and the universal primers in the 3' end, here described as M13 and T7. In this example, the oligonucleotide containing the sequence T7Universal Primer_Barcode_A-Adapter is, but is not restricted to, the sequence that must be present in the collection tube. Additionally, for this system, it is necessary to use an oligonucleotide synthesized as P1_universal primer, here exemplified as P1_M13, in order to have a feasible NGS sequencing target region fragment. The P1_M13 primer was added only at the PCR mix preparation, concomitantly with specific primers containing M13 and T7 sequences. For bi-directional sequencing, in this example, one should consider the use of adapters with M13 and T7 exchanged in described sequences.

At this moment, and for this example, one can expect the following fragments for the PGM system sequencing:
P1_M13_SpecificF_TARGET_REGION_SpecificR_T7_BARCODE_A_Adapter
P1_T7_SpecificF_TARGET_REGION_SpecificR_M13_BARCODE_A_Adapter
P1_M13_SpecificR_TARGET_REGION_SpecificF_T7_BARCODE_A_Adapter
P1_T7_SpecificR_TARGET_REGION_SpecificF_M13_BARCODE_A_Adapter

After these steps target regions are treated as described in NGS workflow protocols. A simplified overview of the entire processes is shown in figure 3.

Table 1 shows examples of the oligonucleotides sequences used in the NGS methodologies described in this document. Specific primers sequences were designed to anneal to a portion of the human *HFE* gene.

**Table1**

| | |
|---|---|
| M13Hemoc**F** specifc | 5'TGTAAAACGACGGCCAGTCTGGATAACCTTGGCTGTACC3' |
| M13Hemoc**R** specifc | 5'TGTAAAACGACGGCCAGTGGCTCTCATCAGTCACATACC3' |
| T7Hemoc**F** specifc | 5'TAATACGACTCACTATAGGGCTGGATAACCTTGGCTGTACC3' |
| T7Hemoc**R** specifc | 5'TAATACGACTCACTATAGGG GGCTCTCATCAGTCACATACC3' |
| P1M13 | 5'CCTCTCTATGGGCAGTCGGTGATTGTAAAACGACGGCCAGT3' |
| P1T7 | 5'CCTCTCTATGGGCAGTCGGTGATTAATACGACTCACTATAGGG3' |
| AdapA_BC1_M13 | 5'CCATCTCATCCCTGCGTGTCTCCGACTCAGACTCACGATATGT AAAACGACGGCCAGT3' |
| AdapA_BC1_T7 | 5'CCATCTCATCCCTGCGTGTCTCCGACTCAGACTCACGATATAA TACGACTCACTATAGGG3' |
| AdapA_BC2_M13 | 5'CCATCTCATCCCTGCGTGTCTCCGACTCAGCGTGTCGCACTGT A AAACGACGGCCAGT3' |
| AdapA_BC2_T7 | 5'CCATCTCATCCCTGCGTGTCTCCGACTCAGCGTGTCGCACTAA T ACGACTCACTATAGGG3' |

### Reaction Conditions

A series of temperatures, enzymes and buffers were evaluated in order to achieve the amplification of a single genome region containing the molecular barcodes. The examples disclosed in the present invention are for illustrative purposes only and do not limit the present disclosure in any way.

In a preferred embodiment the annealing temperature for amplification and barcode insertion is between 35°C to 80°C. In a most preferred embodiment the annealing temperature is between 45°C and 70°C. In an even more preferred embodiment the annealing temperature is 56°C.

In a preferred embodiment the number of cycles of the one step PCR is between 10 and 60 cycles. In a most preferred embodiment the number of cycles is between 30 and 50 cycles. In an even more preferred embodiment the number of cycles is 45.

In a preferred embodiment for a template it is used a pre-prepared mix from PROMEGA. Each reaction will contain two pairs of primers. In this example, one pair (M13 specific primer F + T7_specific primer R) will amplify the target region and the second pair, in this example, (Barcode_M13 + Barcode_T7) will insert the barcode sequences. These two actions are performed with the same annealing temperature and in one step. Figure 2 exemplifies this embodiment.

In a further preferred embodiment similar strategy of the above embodiment was used, however it was used a HotStarTaq (QIAGEN) reagents. Analogous conditions were used to prepare templates for NGS; however, this sequencing methodology use adapters and oligonucleotides as described above and in Figure 3.

In the following examples two distinct reagents systems are used for amplification: (1) HotStarTaq enzyme (QIAGEN 203205 and the other components of the kit; (2) PROMEGA PCR MASTER MIX (catalog M7501). The parameters for each application are described in the respective sections.

The following examples are for illustrative purposes only and do not limit the present disclosure in any way.

### Example 1

A sample consisting of 2µl of blood was transferred to a sample tube comprising anticoagulant (1µl) and barcodes (0,2µl in concentration of 100µM). Then, 20µl of lyse reagent of the Kit TaqMan Sample to SNP were added to the collection tube, followed by mixing the components in a vortex at room temperature for 5 minutes. Finally, 20µl of DNA stabilization reagent of the Kit TaqMan Sample to SNP were also added to the sample tube.

Next, an aliquot of 10µl of the resultant solution were removed and used in the amplification step of the target region in the *HFE gene*.

| | 1X |
|---|---|
| MIX_PROMEGA (2X) | 25 µl |
| Specific Primer F (20uM) | 0,3 µl |
| Specific Primer R (20uM) | 0,3 µl |
| Sample aliquot | 10 µl |
| DNAse/RNAse-Free water | Volume sufficient to 50 µl |

The specific primers used in the amplification of a region of *HFE* gene, in the chromosome 6, to detect the alteration c.845G>A (C282Y). were: **F** 5'CTGGATAACCTTGGCTGTACC3' and **R** 5'GGCTCTCATCAGTCAC ATACC3'. These specific primers were designed so that in the end 5' the sequence of the universal M13 (5'TGTAAAACGACGGCCAGT3') or T7 (5'TAATACGACTCACTATAGGG3') were inserted.

In this step, the difference between the preparation for Sanger or NGS methods relies in the use of oligonucleotides containing adapters ("P1" and "A") for the exemplified NGS method.

The amplification was performed in the thermocycler Veriti (Applied Biosystems - Life Technologies) and the cycling was the following:
- 95°C - 15min
- 95°C - 30seg / 56°C - 30seg / 72°C - 30seg (45 cycles)
- 72°C - 10min
- 4°C - undetermined

After the amplification, the PCR products were purified using the GFX Kit (GE Healthcare 28903471) and quantified by spectrophotometry using Nanodrop 2000 (ThermoScientific). It is important to note that, depending on the particular optimization approaches, this step is optional.

For applications of Sanger sequencing it was used a standard protocol described in the kit BigDye Terminator v3.1 (Life Technologies), available on the company website and for NGS applications was used standard protocol for sequencing of target regions according to Ion PGM (Life Technologies).

### Example 2

A sample consisting of 2µl of blood was transferred to a sample tube comprising anticoagulant (1µl) and barcodes (0,2µl in concentration of 100µM). Then, 20µl of lyse reagent of the Kit TaqMan Sample to SNP were added to the collection tube, followed by mixing the components in a vortex at room temperature for 5 minutes. Finally, 20µl of DNA stabilization reagent of the Kit TaqMan Sample to SNP were also added to the sample tube.

Next, an aliquot of 1µl of the resultant solution were removed and used in the amplification step of the target region in the *JAK2* gene.

| | 1X |
|---|---|
| Buffer (5X) | 5µl |
| dNTP Roche 12269023 (10mM) | 1µl |
| QSolution (5X) | 5µl |
| Specific Primer F (20µM) | 0,3µl |
| Specific Primer R(20µM) | 0,3µl |
| Taq 5U/µl | 0,5µl |
| Sample aliquot | 10µl |
| DNAse/RNAse-Free water | Volume sufficient to 50µl |

The specific primers used in the amplification of a region of *JAK2* gene, in the chromosome 9, to detect the alteration c. c.1849G>T (V617F) were: **F** 5'GCAGCAAGTATGATGAGCAAGCTT3' and **R** 5'GGCATTAGAAAGCCTGTAGTTTTACTTAC3' . These specific primers were designed so that in the end 5' the sequence of the universal M13 (5'TGTAAAACGACGGCCAGT3') or T7 (5'5TAATACGACTCACTATAGGG3') were inserted.

In this step, the difference between the preparation for Sanger or NGS methods relies in the use of oligonucleotides containing adapters ("P1" and "A") for the exemplified NGS method.The amplification was performed in the thermocycler Veriti (Applied Biosystems - Life Technologies) and the cycling was the following:
- 95°C - 5min
- 95°C - 30seg / 56°C - 30seg / 72°C - 30seg (45 cycles)
- 72°C - 10min
- 4°C - undetermined

After the amplification, the PCR products were purified using the GFX Kit (GE Healthcare 28903471) and quantified by spectrophotometry using Nanodrop 2000 (ThermoScientific). It is important to note that, depending on the particular optimization approaches, this step is optional.

For applications of Sanger sequencing, it was used standard protocol described in the kit BigDye Terminator v3.1 (Life Technologies), available on the company website; and for NGS applications, it was used standard protocol for sequencing of target regions according to Ion PGM (Life Technologies).

## Claims

1. A method for complete tracking of a set of biological samples containing DNA or RNA through the use of molecular barcode identification during the course of laboratorial workflow, **characterized by** comprising the following steps:
i) contacting a first pair of oligonucleotide primers, each comprising the same molecular barcode bound to a universal primer, with each biological sample containing DNA or RNA in a collection mean, thus forming mix A;
wherein said primers are already present in the collection mean in which the biological sample was collected, or
wherein the contacting step i) is carried out immediately after said biological sample was collected in said collection mean;
ii) contacting mix A with a PCR mix, thus forming mix B, wherein the PCR mix comprises a second pair of oligonucleotide primers, each comprising a specific primer bound to an universal primer region, wherein the specific primers are specific for a target region of said biological sample and the universal primer regions are complementary to the universal primers of the oligonucleotides of mix A;
iii) amplifying the target region of the biological samples containing DNA or RNA and inserting the molecular barcode in both ends of said target region in a single and concomitant step by performing a multiplex one-step PCR in mix B using the same annealing temperature for amplification and for barcode insertion;
iv)analyzing the product obtained in step iii) by any molecular technology.

2. The method according to claim 1, **characterized in that**, in step i), the molecular barcodes are synthesized, or generated through molecular biology methodologies, in tandem with the universal primers.

3. The method according to claim 2, **characterized in that** the molecular barcodes are from 4 to 30 nucleotides long, preferably composed between 8 to 12 nucleotides and most preferably 10 nucleotides long.

4. The method according to any one of claims 1 to 3, **characterized in that** the volume of said biological samples containing DNA or RNA is from 0,5 µL to 5 mL, more preferably from 1 µL to 10 µL and most preferably 2 µL.

5. The method according to claim 4, **characterized in that** the annealing temperature is between 35°C to 80°C, more preferably between 45°C and 70°C, and most preferably 56°C.

6. The method according to any one of claims 1 to 5, **characterized in that** the number of cycles of the multiplex one-step PCR of step iii) is between 10 and 60 cycles, more preferably between 20 and 50 cycles, and most preferably 45 cycles.

7. The method according to any one of claims 1 to 6, **characterized in that** it is useful for a series of different biological samples from human or any other eukaryotic and prokaryotic organisms selected from the group consisting of blood and other fluids, paraffin or other forms of fixed tissues, cryopreserved samples, cultured cells, tissues, seeds, leaves, exudates, lavages and swabs material.

8. The method according to claim 7, **characterized in that** the biological sample containing DNA or RNA is blood.

9. The method according to claim 8, **characterized in that**, the blood is contacted with anticoagulants selected from the group consisting of EDTA, heparin or citrated-based anticoagulants.

10. The method according to any one of claims 1 to 9, **characterized in that** it is useful for a series of different synthetic samples that can be produced through in vivo or in vitro strategies.

11. The method according to any one of claims 1 to 10, **characterized in that** the universal primers of steps i) and li) are universal primers M13 and T7.

12. The method according to any one of claims 1 to 11, **characterized in that** the molecular technology is a sequencing methodology.

13. The method according to claim 12, **characterized in that** the sequencing methodology is Sanger or Next-Generation Sequencing.

14. The method according to any one of claims 1 to 13, **characterized in that** the collection means is selected from the group consisting of tubes, recipients, plates, papers, swabs and pipettes.

15. The method according to anyone of claims 1 to 14, **characterized in that** steps i) and ii) are carried out simultaneously.

16. The method according to anyone of claims 1 to 14, **characterized in that** step ii) is carried out after step i) in a reaction tube or recipient.

17. A method for complete tracking of a set of biological samples containing DNA or RNA through the use of molecular barcode identification over the course of laboratorial workflow, **characterized by** comprising the following steps:
i) contacting a first pair of oligonucleotide primers, each comprising the same molecular barcode bound to a universal primer, with each biological sample containing DNA or RNA in a collection mean, thus forming a mix A,
wherein said primers are already present in the collection mean in which the biological sample was collected, or
wherein the contacting step i) is carried out immediately after said biological was collected in said collection mean;
ii) transferring an aliquot of mix A to a reaction tube or recipient wherein said reaction tube or recipient comprises a PCR mix, thus forming mix B, wherein the PCR mix comprises a second pair of oligonucleotide primers, each comprising a specific primer bound to an universal primer region, wherein the specific primers are specific for a target region of said biological sample and the universal primer regions are complementary to the universal primers of the oligonucleotides of mix A;
iii) amplifying the target region of the biological samples containing DNA or RNA and inserting the molecular barcodes in both ends of said target region in a single and concomitant step by performing a multiplex one-step PCR in mix B using the same annealing temperature for amplification and for barcode insertion;
iv)analyzing the product obtained in step iii) by any molecular technology.

18. A method for complete tracking of a set of biological samples containing DNA or RNA through the use of molecular barcode identification over the course of laboratorial workflow, **characterized by** comprising the following steps:
i) adding each of the biological samples containing DNA or RNA to a collection mean containing a first pair of oligonucleotide primers, each comprising the same molecular barcode bound to a universal primer, thus forming a mix A;
ii) transferring an aliquot of mix A to a reaction tube or recipient wherein said reaction tube or recipient comprises a PCR mix, thus forming mix B, wherein the PCR mix comprises a second pair of oligonucleotide primers, each comprising specific primers bound to an universal primer region, wherein the specific primers are specific to a target region of said biological sample and the universal primer regions are complementary to the universal primers of the oligonucleotides of mix A;
iii) amplifying the target region of the biological samples containing DNA or RNA and inserting the molecular barcodes in both ends of said target region in a single and concomitant step by performing a multiplex one-step PCR in mix B using the same annealing temperature for amplification and for barcode insertion;
iv) analyzing the product obtained in step iii) by any molecular technology.

19. Use of a kit for performing the method as defined in claim 15, wherein the kit comprises a set of collection tubes or recipients comprising a first pair of oligonucleotide primers, each comprising a molecular barcode bound to a universal primer, and a second pair of oligonucleotide primers, each comprising a specific primer bound to a universal primer region and PCR mix in each collection tube or recipient.

20. Use of a kit for performing the method as defined in claim 17 or 18, wherein the kit comprises a first set of collection tubes or recipients comprising a first pair of oligonucleotide primers, each comprising a molecular barcode bound to a universal primer in each collection tube or recipient, and a second set of reaction tubes or recipients comprising a second pair of oligonucleotide primers, each comprising a specific primer bound to a universal primer region and PCR mix in each reaction tube or recipient.

## Patentansprüche

1. Verfahren zur vollständigen Verfolgung eines Satzes von biologischen Proben mit DNA oder RNA durch die Verwendung von molekularer Strichcodeidentifikation während eines Laborarbeitsablaufs, **dadurch gekennzeichnet, dass** es die folgenden Schritte umfasst:
i) Inkontaktbringen eines ersten Paares von Oligonukleotidprimern, die jeweils denselben molekularen Strichcode umfassen, der mit einem universellen Primer verbunden ist, wobei jede biologische Probe DNA oder RNA in einem Sammelmittel enthält, wodurch Gemisch A gebildet wird;
wobei die Primer bereits in dem Sammelmittel vorhanden sind, in dem die biologische Probe gesammelt wurde, oder wobei der Schritt des Inkontaktbringens i), unmittelbar nachdem die biologische Probe in dem Sammelmittel gesammelt wurde, durchgeführt wird;
ii) Inkontaktbringen von Gemisch A mit einem PCR-Gemisch, wodurch Gemisch B gebildet wird, wobei das PCR-Gemisch ein zweites Paar von Oligonukleotidprimern umfasst, die jeweils einen spezifischen Primer umfassen, der mit einer universellen Primerregion verbunden ist, wobei die spezifischen Primer spezifisch für eine Zielregion der biologischen Probe sind und die universellen Primerregionen komplementär zu den universelle Primern der Oligonukleotide von Gemisch A sind;
iii) Amplifizieren der Zielregion der biologischen Proben mit DNA oder RNA und Einfügen des molekularen Strichcodes in beide Enden der Zielregion in einem einzigen und gleichzeitigen Schritt durch Durchführen einer Multiplex-Einschritt-PCR in Gemisch B unter Verwendung derselben Anlagerungstemperatur zur Amplifikation und zur Strichcodeinsertion;
iv) Analysieren des in Schritt iii) erhaltenen Produkts durch eine beliebige molekulare Technik.

2. Verfahren nach Anspruch 1, **dadurch gekennzeichnet, dass**, in Schritt i), die molekularen Strichcodes durch molekularbiologische Methoden synthetisiert oder erzeugt werden, zusammen mit den universellen Primern.

3. Verfahren nach Anspruch 2, **dadurch gekennzeichnet, dass** die molekularen Strichcodes von 4 bis 30 Nukleotide lang sind, bevorzugt aus zwischen 8 und 12 Nukleotiden bestehen und am stärksten bevorzugt 10 Nukleotide lang sind.

4. Verfahren nach einem der Ansprüche 1 bis 3, **dadurch gekennzeichnet, dass** das Volumen der biologischen Proben mit DNA oder RNA von 0,5 µl bis 5 ml beträgt, stärker bevorzugt von 1 µl bis 10 µl und am stärksten bevorzugt 2 µl.

5. Verfahren nach Anspruch 4, **dadurch gekennzeichnet, dass** die Anlagerungstemperatur zwischen 35 °C und 80 °C liegt, stärker bevorzugt zwischen 45 °C und 70 °C, und am stärksten bevorzugt 56 °C beträgt.

6. Verfahren nach einem der Ansprüche 1 bis 5, **dadurch gekennzeichnet, dass** die Anzahl der Zyklen der Multiplex-Einschritt-PCR von Schritt iii) zwischen 10 und 60 Zyklen liegt, stärker bevorzugt zwischen 20 und 50 Zyklen, und am stärksten bevorzugt 45 Zyklen beträgt.

7. Verfahren nach einem der Ansprüche 1 bis 6, **dadurch gekennzeichnet, dass** es geeignet ist für eine Reihe von verschiedenen biologischen Proben, aus humanen oder beliebigen anderen eukaryontischen und prokaryontischen Organismen, die ausgewählt sind aus der Gruppe, die aus Blut und anderen Flüssigkeiten, Paraffin oder anderen Formen von fixierten Geweben, kryokonservierten Proben, kultivierten Zellen, Geweben, Samen, Blättern, Exsudaten, Lavagen- und Abstrichmaterial besteht.

8. Verfahren nach Anspruch 7, **dadurch gekennzeichnet, dass** die biologische Probe mit DNA oder RNA Blut ist.

9. Verfahren nach Anspruch 8, **dadurch gekennzeichnet, dass** das Blut mit Antikoagulanzien in Kontakt gebracht wird, die aus der Gruppe ausgewählt sind, die aus EDTA, Heparin oder citratbasierten Antikoagulanzien besteht.

10. Verfahren nach einem der Ansprüche 1 bis 9, **dadurch gekennzeichnet, dass** es für eine Reihe von verschiedenen synthetischen Proben geeignet ist, die mittels In-vivo- oder In-vitro-Strategien hergestellt werden können.

11. Verfahren nach einem der Ansprüche 1 bis 10, **dadurch gekennzeichnet, dass** die universellen Primer von Schritt i) und ii) die universellen Primer M13 und T7 sind.

12. Verfahren nach einem der Ansprüche 1 bis 11, **dadurch gekennzeichnet, dass** die molekulare Technik eine Sequenziermethode ist.

13. Verfahren nach Anspruch 12, **dadurch gekennzeichnet, dass** die Sequenziermethode Sanger-Sequenzierung oder Next-Generation Sequencing ist.

14. Verfahren nach einem der Ansprüche 1 bis 13, **dadurch gekennzeichnet, dass** das Sammelmittel aus der Gruppe ausgewählt ist, die aus Röhrchen, Empfängern, Platten, Papieren, Tupfern und Pipetten besteht.

15. Verfahren nach einem der Ansprüche 1 bis 14, **dadurch gekennzeichnet, dass** die Schritte i) und ii) gleichzeitig durchgeführt werden.

16. Verfahren nach einem der Ansprüche 1 bis 14, **dadurch gekennzeichnet, dass** Schritt ii) nach Schritt i) in einem Reaktionsröhrchen oder Empfänger durchgeführt wird.

17. Verfahren zur vollständigen Verfolgung eines Satzes von biologischen Proben mit DNA oder RNA durch die Verwendung von molekularer Strichcodeidentifikation während eines Laborarbeitsablaufs, **dadurch gekennzeichnet, dass** es die folgenden Schritte umfasst:
i) Inkontaktbringen eines ersten Paares von Oligonukleotidprimern, die jeweils denselben molekularen Strichcode umfassen, der mit einem universellen Primer verbunden ist, wobei jede biologische Probe DNA oder RNA in einem Sammelmittel enthält, wodurch Gemisch A gebildet wird;
wobei die Primer bereits in dem Sammelmittel vorhanden sind, in dem die biologische Probe gesammelt wurde, oder wobei der Schritt des Inkontaktbringens i), unmittelbar nachdem die biologische Probe in dem Sammelmittel gesammelt wurde, durchgeführt wird;
ii) Überführen eines Aliquots von Gemisch A in ein Reaktionsröhrchen oder einen Empfänger, wobei das Reaktionsröhrchen oder der Empfänger ein PCR-Gemisch umfasst, wodurch Gemisch B gebildet wird, wobei das PCR-Gemisch ein zweites Paar von Oligonukleotidprimern umfasst, die jeweils einen spezifischen Primer umfassen, der mit einer universellen Primerregion verbunden ist, wobei die spezifischen Primer spezifisch für eine Zielregion der biologischen Probe sind und die universellen Primerregionen komplementär zu den universellen Primern der Oligonukleotide von Gemisch A sind;
iii) Amplifizieren der Zielregion der biologischen Proben mit DNA oder RNA und Einfügen der molekularen Strichcodes in beide Enden der Zielregion in einem einzigen und gleichzeitigen Schritt durch Durchführen einer Multiplex-Einschritt-PCR in Gemisch B unter Verwendung derselben Anlagerungstemperatur zur Amplifikation und zur Strichcodeinsertion;
iv) Analysieren des in Schritt iii) erhaltenen Produkts durch eine beliebige molekulare Technik.

18. Verfahren zur vollständigen Verfolgung eines Satzes von biologischen Proben mit DNA oder RNA durch die Verwendung von molekularer Strichcodeidentifikation während eines Laborarbeitsablaufs, **dadurch gekennzeichnet, dass** es die folgenden Schritte umfasst:
i) Zufügen von jeder der biologischen Proben mit DNA oder RNA zu einem Sammelmittel, das ein erstes Paar von Oligonukleotidprimern enthält, die jeweils denselben molekularen Strichcode umfassen, der mit einem universellen Primer verbunden ist, wodurch ein Gemisch A gebildet wird;
ii) Überführen eines Aliquots von Gemisch A in ein Reaktionsröhrchen oder einen Empfänger, wobei das Reaktionsröhrchen oder der Empfänger ein PCR-Gemisch umfasst, wodurch Gemisch B gebildet wird, wobei das PCR-Gemisch ein zweites Paar von Oligonukleotidprimern umfasst, die jeweils spezifische Primer umfassen, die mit einer universellen Primerregion verbunden sind, wobei die spezifischen Primer spezifisch für eine Zielregion der biologischen Probe sind und die universellen Primerregionen komplementär zu den universellen Primern der Oligonukleotide von Gemisch A sind;
iii) Amplifizieren der Zielregion der biologischen Proben mit DNA oder RNA und Einfügen der molekularen Strichcodes in beide Enden der Zielregion in einem einzigen und gleichzeitigen Schritt durch Durchführen einer Multiplex-Einschritt-PCR in Gemisch B unter Verwendung derselben Anlagerungstemperatur zur Amplifikation und zur Strichcodeinsertion;
iv) Analysieren des in Schritt iii) erhaltenen Produkts durch eine beliebige molekulare Technik.

19. Verwendung eines Kits zum Durchführen des wie in Anspruch 15 definierten Verfahrens, wobei das Kit einen Satz an Sammelröhrchen oder Empfängern umfasst, die ein erstes Paar von Oligonukleotidprimern umfassen, die jeweils einen molekularen Strichcode umfassen, der mit einem universellen Primer verbunden ist, und ein zweites Paar von Oligonukleotidprimern, die jeweils einen spezifischen Primer umfassen, der mit einer universellen Primerregion verbunden ist, und PCR-Gemisch in jedem Sammelröhrchen oder Empfänger.

20. Verwendung eines Kits zum Durchführen des wie in Anspruch 17 oder 18 definierten Verfahrens, wobei das Kit einen ersten Satz an Sammelröhrchen oder Empfängern umfasst, die in jedem Sammelröhrchen oder Empfänger ein erstes Paar von Oligonukleotidprimern umfassen, die jeweils einen molekularen Strichcode umfassen, der mit einem universellen Primer verbunden ist, und einen zweiten Satz an Reaktionsröhrchen oder Empfängern, die in jedem Reaktionsröhrchen oder Empfänger ein zweites Paar von Oligonukleotidprimern, die jeweils einen spezifischen Primer umfassen, der mit einer universellen Primerregion verbunden ist, und PCR-Gemisch umfassen.

## Revendications

1. Méthode de suivi complet d'un set d'échantillons biologiques contenant de l'ADN ou de l'ARN via l'utilisation d'une identification par code-barres moléculaire au cours d'un rythme de travail de laboratoire, **caractérisée en ce qu'**elle comprend les étapes suivantes :
i) la mise en contact d'une première paire d'amorces oligonucléotidiques, comprenant chacune le même code-barres moléculaire lié à une amorce universelle, avec chaque échantillon biologique contenant de l'ADN ou de l'ARN dans un moyen de collecte, formant ainsi un mélange A ;
où lesdites amorces sont déjà présentes dans le moyen de collecte dans lequel l'échantillon biologique a été collecté, ou
où l'étape de mise en contact i) est effectuée immédiatement après la collecte dudit échantillon biologique dans ledit moyen de collecte ;
ii) la mise en contact du mélange A avec un mélange pour PCR, formant ainsi un mélange B, où le mélange pour PCR comprend une deuxième paire d'amorces oligonucléotidiques, comprenant chacune une amorce spécifique liée à une région d'amorce universelle, où les amorces spécifiques sont spécifiques d'une région cible dudit échantillon biologique et les régions d'amorce universelle sont complémentaires des amorces universelles des oligonucléotides du mélange A ;
iii) l'amplification de la région cible des échantillons biologiques contenant de l'ADN ou de l'ARN et l'insertion du code-barres moléculaire aux deux extrémités de ladite région cible dans une étape unique et concomitante par la mise en oeuvre d'une PCR multiplex à une étape dans le mélange B en utilisant la même température d'annelage pour l'amplification et pour l'insertion du code-barres ;
iv) l'analyse du produit obtenu dans l'étape iii) par une technologie moléculaire quelconque.

2. Méthode selon la revendication 1, **caractérisée en ce que**, dans l'étape i), les codes-barres moléculaires sont synthétisés ou générés par des méthodologies de biologie moléculaire, en tandem avec les amorces universelles.

3. Méthode selon la revendication 2, **caractérisée en ce que** les codes-barres moléculaires sont d'une longueur allant de 4 à 30 nucléotides, préférablement composés de 8 à 12 nucléotides et tout préférablement d'une longueur de 10 nucléotides.

4. Méthode selon l'une quelconque des revendications 1 à 3, **caractérisée en ce que** le volume desdits échantillons biologiques contenant de l'ADN ou de l'ARN va de 0,5 µl à 5 ml, plus préférablement de 1 µl à 10 µl et tout préférablement est de 2 µl.

5. Méthode selon la revendication 4, **caractérisée en ce que** la température d'annelage va de 35°C à 80°C, plus préférablement est comprise entre 45°C et 70°C, et tout préférablement est de 56°C.

6. Méthode selon l'une quelconque des revendications 1 à 5, **caractérisée en ce que** le nombre de cycles de la PCR multiplex à une étape de l'étape iii) est compris entre 10 et 60 cycles, plus préférablement entre 20 et 50 cycles, et tout préférablement est de 45 cycles.

7. Méthode selon l'une quelconque des revendications 1 à 6, **caractérisée en ce qu'**elle est utile pour une série d'échantillons biologiques différents issus d'organismes humains ou autres eucaryotes ou procaryotes quelconques, choisis dans le groupe constitué par le sang et autres fluides, la paraffine et d'autres formes de tissus fixés, les échantillons en cryoconservation, les cellules en culture, les tissus, les graines, les feuilles, les matériaux issus d'exsudats, de lavements et de prélèvements.

8. Méthode selon la revendication 7, **caractérisée en ce que** l'échantillon biologique contenant de l'ADN ou de l'ARN est du sang.

9. Méthode selon la revendication 8, **caractérisée en ce que** le sang est mis en contact avec des anticoagulants choisis dans le groupe constitué par l'EDTA, l'héparine ou des anticoagulants à base de citrate.

10. Méthode selon l'une quelconque des revendications 1 à 9, **caractérisée en ce qu'**elle est utile pour une série d'échantillons synthétiques différents pouvant être produits par des stratégies *in vivo* ou *in vitro.*

11. Méthode selon l'une quelconque des revendications 1 à 10, **caractérisée en ce que** les amorces universelles des étapes i) et ii) sont les amorces universelles M13 et T7.

12. Méthode selon l'une quelconque des revendications 1 à 11, **caractérisée en ce que** la technologie moléculaire est une méthodologie de séquençage.

13. Méthode selon la revendication 12, **caractérisée en ce que** la méthodologie de séquençage est un Séquençage de Sanger ou de Nouvelle Génération.

14. Méthode selon l'une quelconque des revendications 1 à 13, **caractérisée en ce que** le moyen de collecte est choisi dans le groupe constitué par les tubes, les récipients, les plaques, les papiers, les écouvillons et les pipettes.

15. Méthode selon l'une quelconque des revendications 1 à 14, **caractérisée en ce que** les étapes i) et ii) sont effectuées simultanément.

16. Méthode selon l'une quelconque des revendications 1 à 14, **caractérisée en ce que** l'étape ii) est effectuée après l'étape i) dans le tube ou le récipient de réaction.

17. Méthode de suivi complet d'un set d'échantillons biologiques contenant de l'ADN ou de l'ARN via l'utilisation d'une identification par code-barres moléculaire au cours d'un rythme de travail de laboratoire, **caractérisée en ce qu'**elle comprend les étapes suivantes :
i) la mise en contact d'une première paire d'amorces oligonucléotidiques, comprenant chacune le même code-barres moléculaire lié à une amorce universelle, avec chaque échantillon biologique contenant de l'ADN ou de l'ARN dans un moyen de collecte, formant ainsi un mélange A ;
où lesdites amorces sont déjà présentes dans le moyen de collecte dans lequel l'échantillon biologique a été collecté, ou
où l'étape de mise en contact i) est effectuée immédiatement après la collecte dudit échantillon biologique dans ledit moyen de collecte ;
ii) le transfert d'une aliquote de mélange A dans un tube ou un récipient de réaction, où ledit tube ou récipient de réaction comprend un mélange pour PCR, formant ainsi un mélange B, où le mélange pour PCR comprend une deuxième paire d'amorces oligonucléotidiques, comprenant chacune une amorce spécifique liée à une région d'amorce universelle, où les amorces spécifiques sont spécifiques d'une région cible dudit échantillon biologique et les régions d'amorce universelle sont complémentaires des amorces universelles des oligonucléotides du mélange A ;
iii) l'amplification de la région cible des échantillons biologiques contenant de l'ADN ou de l'ARN et l'insertion du code-barres moléculaire aux deux extrémités de ladite région cible dans une étape unique et concomitante par la mise en oeuvre d'une PCR multiplex à une étape dans le mélange B en utilisant la même température d'annelage pour l'amplification et pour l'insertion du code-barres ;
iv) l'analyse du produit obtenu dans l'étape iii) par une technologie moléculaire quelconque.

18. Méthode de suivi complet d'un set d'échantillons biologiques contenant de l'ADN ou de l'ARN via l'utilisation d'une identification par code-barres moléculaire au cours d'un rythme de travail de laboratoire, **caractérisée en ce qu'**elle comprend les étapes suivantes :
i) l'addition de chacun parmi les échantillons biologiques contenant de l'ADN ou de l'ARN à un moyen de collecte contenant une première paire d'amorces oligonucléotidiques, comprenant chacune le même code-barres moléculaire lié à une amorce universelle, formant ainsi un mélange A ;
ii) le transfert d'une aliquote de mélange A dans un tube ou un récipient de réaction, où ledit tube ou récipient de réaction comprend un mélange pour PCR, formant ainsi un mélange B, où le mélange pour PCR comprend une deuxième paire d'amorces oligonucléotidiques, comprenant chacune des amorces spécifiques liées à une région d'amorce universelle, où les amorces spécifiques sont spécifiques d'une région cible dudit échantillon biologique et les régions d'amorce universelle sont complémentaires des amorces universelles des oligonucléotides du mélange A ;
iii) l'amplification de la région cible des échantillons biologiques contenant de l'ADN ou de l'ARN et l'insertion des codes-barres moléculaires aux deux extrémités de ladite région cible dans une étape unique et concomitante par la mise en oeuvre d'une PCR multiplex à une étape dans le mélange B en utilisant la même température d'annelage pour l'amplification et pour l'insertion du code-barres ;
iv) l'analyse du produit obtenu dans l'étape iii) par une technologie moléculaire quelconque.

19. Utilisation d'un kit destiné à mettre en oeuvre la méthode telle que définie selon la revendication 15, dans laquelle le kit comprend un set de tubes ou de récipients de collecte comprenant une première paire d'amorces oligonucléotidiques, comprenant chacune un code-barres moléculaire lié à une amorce universelle, et une deuxième paire d'amorces oligonucléotidiques, comprenant chacune une amorce spécifique liée à une région d'amorce universelle et un mélange pour PCR dans chaque tube ou récipient de collecte.

20. Utilisation d'un kit destiné à mettre en oeuvre la méthode telle que définie selon la revendication 17 ou 18, dans laquelle le kit comprend un premier set de tubes ou de récipients de collecte comprenant une première paire d'amorces oligonucléotidiques, comprenant chacune un code-barres moléculaire lié à une amorce universelle dans chaque tube ou récipient de collecte, et un deuxième set de tubes ou de récipients de réaction comprenant une deuxième paire d'amorces oligonucléotidiques, comprenant chacune une amorce spécifique liée à une région d'amorce universelle et un mélange pour PCR dans chaque tube ou récipient de réaction.
